# FASCICULE DE BREVET EUROPEEN

(11) **EP 0 861 818 B1**
(45) Date de publication et mention de la délivrance du brevet: **27.12.2000**
(21) Numéro de dépôt: 98400385.5
(22) Date de dépôt: 18.02.1998
(51) Int. Cl.: C07C 11/02, C07C 1/20

(54) **Procédé de production d'oléfine tertiaire par décomposition d'éther alkylique tertiaire comportant une première étape de purification par lavage à l'eau**
Verfahren zur Herstellung von tertiären Olefinen durch Spaltung von tertiären Alkylethern in dem die erste Reinigungsstufe eine Wäsche mit Wasser ist
Process for the preparation of tertiary olefins by decomposition of tertiary alkyl ethers comprising a water wash as first purification step

(30) Priorité: 26.02.1997 FR 9702390
(43) Date de publication de la demande: 02.09.1998
(73) Titulaire: INSTITUT FRANCAIS DU PETROLE, 92852 Rueil-Malmaison Cedex (FR)
(72) Inventeur: Marion, Marie-Claire, 69100 Villeurbanne (FR); Coupard, Vincent, 69002 Lyon (FR); Forestiere, Alain, 69390 Vernaison (FR); Travers, Philippe, 92500 Rueil Malmaison (FR); Viltard, Jean-Charles, 26000 Valence (FR)
(74) Mandataire: Andréeff, François

(56) Documents cités:
- US-A- 4 447 668

## Description

L'invention concerne un procédé de décomposition d'éther(s) alkylique(s) tertiaire(s) pour la production d'oléfine(s) tertiaire(s) de haute pureté. Elle concerne en particulier un procédé de production d'isobutène de très haute pureté, et de méthanol, à partir de l'éther méthylique de l'alcool tertio-butylique (MTBE initiales anglaises de Methyl-Tertio-Butyl-Ether). Le procédé de la présente invention s'applique à la synthèse de toute oléfine tertiaire à partir d'éther alkylique tertiaire [par exemple ETBE (éther éthylique de l'alcool tertio-butylique des initiales anglaises Ethyl-Tertio-Butyl-Ether), ETAE (éther méthylique de l'alcool tertioamylique des initiales anglaises Tertio-Amyl-Methyl-Ether), TAME (Ether méthylique de l'alcool tertioamylique des initiales anglaises Tertio-Amyl-Methyl-Ether), isopropyl-tertio-butyl-éther]. La suite de la description et tout particulièrement les conditions opératoires de mise en oeuvre de toute zone est donnée à titre indicatif pour la synthèse d'isobutène à partir de MTBE.

Il existe diverses voies de production d'isobutène de haute pureté exploitées industriellement. La plus ancienne est le procédé par extraction à l'acide sulfurique, mais il est cher et obsolète ; il est réputé polluant car il produit des rejets d'acide usé. De plus, le rendement en isobutène ne dépasse pas 90 %. La firme ARCO utilise la voie par déshydratation de l'alcool tertio-butylique (ABT), ce dernier étant un sous-produit obtenu avec leur procédé de production d'oxyde de propylène. Le procédé par déshydrogénation de l'isobutane s'est développé au cours de ces dernières années par suite de la demande importante et croissante en MTBE. Toutefois, ce procédé n'est mis en oeuvre de façon rentable que pour de très grosses capacités de production.

La production d'isobutène de haute pureté par craquage du MTBE est aussi bien adaptée pour des petites capacités que pour des grosses capacités. De plus, cette voie bénéficie de toute l'infrastructure relative à l'importance croissante des éthers dans les essences reformulées. De nombreuses raffineries, partout dans le monde, possèdent des installations de productions de MTBE par exemple. D'autre part il y a également un marché d'échange du MTBE au niveau mondial. Cela signifie que la production d'isobutène de haute pureté, à partir de MTBE, peut être mise en oeuvre aisément partout dans le monde, y compris en dehors des raffineries.

L'idée de produire de l'isobutène par décomposition d'éther, et plus particulièrement du MTBE, est connue depuis longtemps, mais les procédés de mise en oeuvre proposés par l'art antérieur présentent certains inconvénients.

Ainsi dans le procédé développé par SUMITOMO, décrit par exemple dans la demande de brevet EP-A-68 785, la réaction de décomposition du MTBE est réalisée en phase liquide, en présence d'un catalyseur solide acide de type résine échangeuse d'ions. Deux flux de produit sont obtenus : l'isobutène et le méthanol. Tel que le schéma est décrit, l'isobutène est directement obtenu en tête d'une colonne à distiller sans autre étape de purification. L'isobutène ainsi obtenu contient un certain nombre d'impuretés à commencer par une petite fraction de méthanol qui est distillée par azéotropie, du diméthyléther (DME) composé volatil formé par condensation du méthanol en présence d'un catalyseur acide. Il est probable que la pureté de l'isobutène soit alors insuffisante pour une utilisation telle que la fabrication de polyisobutène ou d'autres copolymères. En outre, aucun moyen ne permet apparemment d'éviter l'accumulation d'impuretés lourdes telles que les dimères de l'isobutène ou l'éther méthylique de l'alcool butylique secondaire (MSBE), qui à terme se traduit fatalement par une baisse de pureté des produits.

Dans le procédé développé par ERDOLCHEMIE, décrit par exemple dans le brevet US-A-4 409 421 la purification de l'isobutène, qui consiste à éliminer l'alcool résiduel entraîné avec l'oléfine tertiaire, est réalisée par adsorption. Cette méthode, présente l'inconvénient d'avoir à régénérer régulièrement l'adsorbant. En outre la récupération de la majeure partie de l'alcool issu de la décomposition n'est pas solutionnée.

Plus récemment, la même société décrit, dans le brevet US-A-5 095 164, la mise en oeuvre de la réaction de décomposition dans un appareillage de distillation. Le catalyseur est alors placé dans le fond de la colonne au niveau du rebouilleur. Cette mise en oeuvre particulière est limitative sur le plan de la température réactionnelle, directement imposée par la nature de l'éther et la pression opératoire. En outre, elle favorise vraisemblablement la formation de sous-produits de réaction tels que la formation de dimères de l'isobutène et/ou la formation de diméthyléther. A ce propos, la qualité et/ou le devenir des produits n'est pas clairement explicité.

Par ailleurs, la société BASF décrit, dans le brevet US-A-4 287 379, un schéma intégrant à la fois l'étape de synthèse de l'éther, sa séparation, puis l'étape de décomposition de l'éther pour produire l'isobutène. Toutefois, pour éviter certaines étapes de purification, l'éthérification est faite avec un alcool en C3 ou C4, ce qui est un inconvénient majeur face au marché international du MTBE.

Nous pouvons également signaler les deux schémas de principe proposés par la société SNAMPROGETTI dans Chemical Economy & Engineering Review, vol. 14 n° 6 Juin 1982, incluant à la fois l'étape de synthèse du MTBE et l'étape de décomposition du MTBE pour la production d'isobutène. Ces schémas mettent en oeuvre une zone de fractionnement par distillation immédiatement après la zone réactionnelle de décomposition. Or, le produit alors traité étant riche en méthanol, l'opération de fractionnement résulte en l'obtention de deux effluents contenant tous les deux ledit alcool : dans l'effluent de tête, l'alcool est entraîné par azéotropie, dans l'effluent de fond on récupère en principe la majorité de l'alcool obtenu dans l'étape de décomposition de l'éther. Dans un tel schéma, la récupération de l'alcool est donc compliquée puisque qu'elle doit être mise en oeuvre à la fois sur l'effluent de tête de la colonne de fractionnement et également sur l'effluent de fond de ladite colonne.

Selon la description de la demande de brevet RO 105954, l'étape de décomposition a lieu dans un réacteur adiabatique en présence de vapeur d'eau. La présence d'eau dans le milieu réactionnel est néfaste à la sélectivité de la réaction de décomposition car il y a perte d'isobutène par réaction avec l'eau pour faire de l'alcool tertio-butylique (ABT). De plus, le système oblige également à prévoir une étape supplémentaire de décantation. En outre, la colonne de récupération de l'alcool (colonne C2 sur la figure du brevet) est énorme compte tenu des quantités d'eau mise en oeuvre.

Dans le procédé décrit dans le brevet US 4,447,668, la réaction de dissociation de l'éther, le MTBE, est suivie d'un lavage à l'eau, puis d'une distillation permettant de séparer l'isobutène récupéré en tête de colonne du MTBE récupéré en fond de colonne. Ce procédé ne prévoit pas la séparation de la phase aqueuse après l'étape de lavage à l'eau.

La demande de brevet WO 91/01804 décrit principalement la possibilité de régénérer le catalyseur, qui est de préférence une argile. Ainsi, l'éther et/ou l'alcool extrait au cours de l'étape de lavage sont renvoyés à la section réactionnelle pour être utilisé comme flux de régénération. Ce système de fonctionnement discontinu, avec alternance de période de réaction et période de régénération, de fréquence soutenue, résulte de l'utilisation d'un catalyseur instable dans le temps. L'exemple unique donné concerne la décomposition de l'éther méthylique de l'alcool tertio-amylique (TAME initiales anglaises de Tertio-Amyl-Methyl-Ether) pour faire des isoamylènes, réaction moins exigeante vis-à-vis de la température que celle de la décomposition du MTBE, au sens de la position de l'équilibre thermodynamique. Il présente alors des difficultés d'opération et également des difficultés d'insertion dans un schéma généralement intégré où les unités annexes fonctionnent en continu (par exemple l'unité d'éthérification, de synthèse du MTBE ou du TAME). Il est alors nécessaire de recourir par exemple à des stockages des produits en amont et en aval, ce qui induit alors des coûts supplémentaires et également un effort de gestion soutenu.

Le procédé selon l'invention permet de remédier aux inconvénients précités des systèmes décrits dans les publications antérieures citées ci-devant. Il concerne un procédé de production d'oléfine(s) tertiaire(s) caractérisée(s) par une très haute pureté, à partir d'un éther alkylique tertiaire et sa mise en oeuvre.

L'invention concerne un procédé de décomposition d'éther(s) alkylique tertiaire(s) en particulier tel(s) que défini(s) précédemment, de préférence de MTBE ou ETBE, pour la production d'oléfine(s) tertiaire(s), d'isobutène en particulier, de haute pureté. Dans le cas de la décomposition d'autres éthers, on peut obtenir un mélange contenant une pluralité d'oléfines tertiaires. Ainsi dans le cas de la décomposition du TAME. On obtient un mélange contenant du méthyl-2 butène-1 et du méthyl-2 butène-2.

Outre la zone de réaction proprement dite, le procédé selon l'invention comprend des zones de purification ou récupération ou recyclage des divers produits de façon à optimiser la valorisation des produits mis en oeuvre et minimiser les pertes.

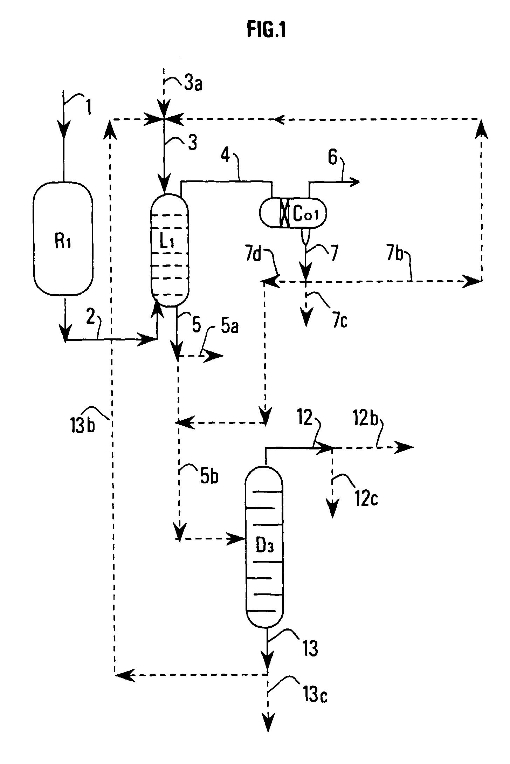

Les figures 1 à 7 sont des schémas de principe illustrants chacun une des multiples variantes de mise en oeuvre du procédé selon la présente invention. Les traits en pointillés montrent les diverses options possibles, tant en ce qui concerne les options de recyclage qu'en ce qui concerne les appareillages optionnels dans la variante considérée. La description du procédé selon la présente invention est faite en liaison avec ces figures dans le but d'en faciliter la compréhension. Sur ces figures, les organes similaires sont désignés par les même chiffres et lettres de référence. Cela ne doit pas être considéré comme une limitation et d'autres variantes non illustrées sont parties intégrantes de la présente invention dans la mesure où elles découlent de façon évidente de la description. La figure 1 qui illustre la forme la plus générale du procédé selon la présente invention est décrite ci-après.

La présente invention concerne un procédé de production d'oléfine tertiaire par décomposition d'éther alkylique tertiaire comprenant
a) une étape a) de décomposition d'au moins un éther alkylique tertiaire dans une zone réactionnelle comprenant au moins un réacteur (R1) contenant un catalyseur de décomposition dudit éther, ladite étape étant effectuée dans des conditions permettant la décomposition au moins partielle dudit éther alkylique tertiaire en un produit (P) contenant au moins un alcool et au moins une oléfine tertiaire et éventuellement de l'éther non décomposé dans cette étape a) et des composés légers éventuels, généralement contenus initialement dans le produit issu de l'étape a),
b) une étape b) de purification d'au moins une partie du produit (P), et de préférence de la totalité de ce produit, dans une zone d'extraction (L1) par lavage à l'eau, à partir de laquelle on obtient une fraction aqueuse (A1) contenant la majeure partie de l'alcool initialement présent dans ladite partie et une fraction (B1) contenant la majeure partie de l'oléfine tertiaire initialement présente dans ladite partie, ladite fraction (B1) contenant ladite oléfine tertiaire, de l'eau, éventuellement de l'éther et des composés légers éventuels et étant sensiblement exempte d'alcool ou en contenant une très faible proportion (par exemple moins de 10%, souvent moins de 5%, et le plus souvent moins de 2 % en poids),
ledit procédé étant caractérisé en ce qu'il comporte une étape c) dans laquelle au moins une partie de la fraction (B1) issue de l'étape b) est envoyée dans une zone de séparation (Co1), de préférence comprenant au moins un dispositif de coalescence ou coalesceur, à partir de laquelle on récupère une fraction liquide aqueuse (La1) et une fraction liquide organique (Lb1) contenant la majeure partie de l'oléfine tertiaire initialement présente dans ladite partie de la fraction (B1), ladite fraction (Lb1) contenant ladite oléfine tertiaire et éventuellement de l'éther et des composés légers éventuels.

Cette forme de mise en oeuvre de la présente invention est en particulier illustrée par la figure 1. La charge contenant 1' (les) éther(s) que l'on souhaite décomposer est introduite dans le réacteur (R1) contenant un catalyseur de décomposition dudit éther, par la ligne 1. On récupère par la ligne 2 un produit (P) contenant au moins un alcool et au moins une oléfine tertiaire et éventuellement de l'éther non décomposé dans cette étape a) et éventuellement des composés légers et on l'envoie dans la zone d'extraction par lavage à l'eau (L1) dans laquelle l'eau nécessaire au lavage est introduite par la ligne 3. A partir de cette zone d'extraction (L1) par lavage à l'eau on obtient par la ligne 5 une fraction aqueuse (A1) contenant de l'eau et la majeure partie de l'alcool initialement présent dans le produit (P) et par la ligne 4 une fraction (B1) contenant la majeure partie de l'oléfine tertiaire initialement présente dans le produit (P) ladite fraction (B1) contenant ladite oléfine tertiaire, de l'eau, éventuellement de l'éther non décomposé dans l'étape a) et éventuellement des composés légers et étant sensiblement exempte d'alcool. Cette fraction (B1) est introduite par la ligne 4 dans un coalesceur (Co1) de l'étape c) à partir duquel on récupère par la ligne 7 une fraction essentiellement aqueuse (La1) et par la ligne 6 une fraction organique (Lb1) contenant de l'oléfine tertiaire purifiée (Ohp1).

Dans le cadre du procédé de la présente invention la fraction aqueuse (A1) peut être récupérée telle quelle pour être envoyée dans une section de traitement des eaux ou être scindée en une fraction aqueuse appauvrie en alcool, par exemple réutilisable comme eau de lavage ou que l'on peut envoyer dans une section de traitement des eaux, et en une fraction organique enrichie en alcool, que l'on peut par exemple récupérer et envoyer dans une zone de synthèse d'éther par réaction entre une oléfine et un alcool.

Le procédé de la présente invention peut comprendre diverses variantes qui permettent en particulier d'améliorer la qualité de l'oléfine tertiaire récupérée et qui sont décrites ci-après. Ces variantes peuvent être réalisées séparément ou simultanément, soit en totalité, soit associées par deux ou plus.

Selon une variante le procédé de la présente invention (voir en particulier la figure 1) comprend un recyclage au moins partiel de la fraction liquide (La1) obtenue à l'étape c) vers la zone d'extraction à l'eau (L1) par les lignes 7, 7b et 3. Il est possible de recycler la totalité de cette fraction (La1) obtenue à l'étape c) vers la zone d'extraction à l'eau (L1) de l'étape b). Lorsque ce recyclage est partiel ou qu'il n'existe pas, une partie ou la totalité de la fraction aqueuse (La1) peut être par exemple envoyée vers une zone de traitement des eaux usées par les lignes 7 et 7c.

Selon une autre variante, le procédé de la présente invention comprend une étape f) (voir en particulier figures 1, 3, 5, 6, 7) dans laquelle au moins une partie, voire la totalité, de ladite fraction (A1) issue de l'étape b) par la ligne 5 est envoyée par la ligne 5b pour la figure 1 dans une zone de fractionnement (D3) à partir de laquelle on récupère par la ligne 12 une fraction (G1) contenant la majeure partie de l'alcool initialement présent dans ladite partie et par la ligne 13 une fraction aqueuse (H1) débarrassée de la majeure partie de l'alcool initialement présent dans ladite partie. Selon ce mode de fonctionnement au moins une partie de la fraction (G1) obtenue à l'étape f) contenant de l'alcool peut être envoyée par la ligne 12c dans une zone de synthèse d'éther par réaction entre au moins une oléfine tertiaire et au moins un alcool. Il est également possible d'envoyer la totalité de cet alcool dans ladite zone de synthèse d'éther. On peut également récupérer par la ligne 12b en partie ou en totalité cet alcool pour d'autres usages. Il est également possible de recycler au moins une partie, voire la totalité, de la fraction aqueuse (H1) par les lignes 13b et 3 vers la zone (L1) d'extraction à l'eau de l'étape b). Lorsque ce recyclage est partiel ou qu'il n'existe pas, cette partie de la fraction aqueuse (H1) peut être par exemple envoyée par la ligne 13c vers une zone de traitement des eaux usées.

Selon une autre variante du procédé selon l'invention (voir en particulier figure 1) au moins une partie de la fraction liquide (La1) obtenue à l'étape c) peut être envoyée par les lignes 7, 7d et 5b dans la zone de fractionnement (D3) de l'étape f).

Dans le cas des variantes ci-devant où l'on recycle de l'eau dans la zone (L1), soit à partir de la fraction (La1) issue de l'étape c), soit à partir de la fraction aqueuse (H1) issue de l'étape f), soit encore à partir de ces deux fractions, la quantité d'eau employée dans la zone d'extraction (L1) est ajustée si nécessaire à l'aide d'au moins un moyen d'introduction d'eau d'appoint par les lignes 3a et 3 dans ladite zone (L1) (voir en particulier figure 1, 3, 5, 6, 7). Cet appoint d'eau permet en particulier de compenser les pertes d'eau par entraînement ou dues à la saturation du flux hydrocarboné traité ainsi que le remplacement de l'eau éventuellement purgée.

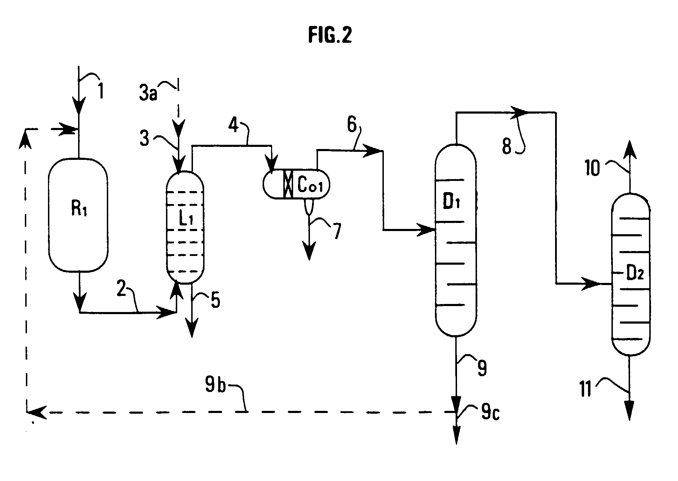

Selon une autre variante, le procédé de la présente invention comprend une étape d) (voir en particulier les figures 2 à 7) dans laquelle au moins une partie de la fraction liquide organique (Lb1) (contenant de l'oléfine tertiaire purifiée (Ohp1)) issue de l'étape c), et de préférence la totalité de ladite fraction, est envoyée par la ligne 6 dans une zone de fractionnement (D1) à partir de laquelle on récupère par la ligne 9 une fraction (E1) contenant la majeure partie de l'éther initialement contenu dans ladite partie et par la ligne 8 une fraction organique (F1) contenant la majeure partie de l'oléfine tertiaire initialement contenue dans ladite partie et éventuellement des composés légers. Ladite fraction (F1) contient de l'oléfine tertiaire purifiée (Ohp2). Selon ce mode de fonctionnement au moins une partie, voire la totalité, de la fraction (E1) peut être recyclée par la ligne 9b dans la zone (R1). Lorsque ce recyclage est partiel ou qu'il n'existe pas, cette partie de la fraction (E1) peut être par exemple envoyée par la ligne 9c au pool essence ou dans une zone de stockage.

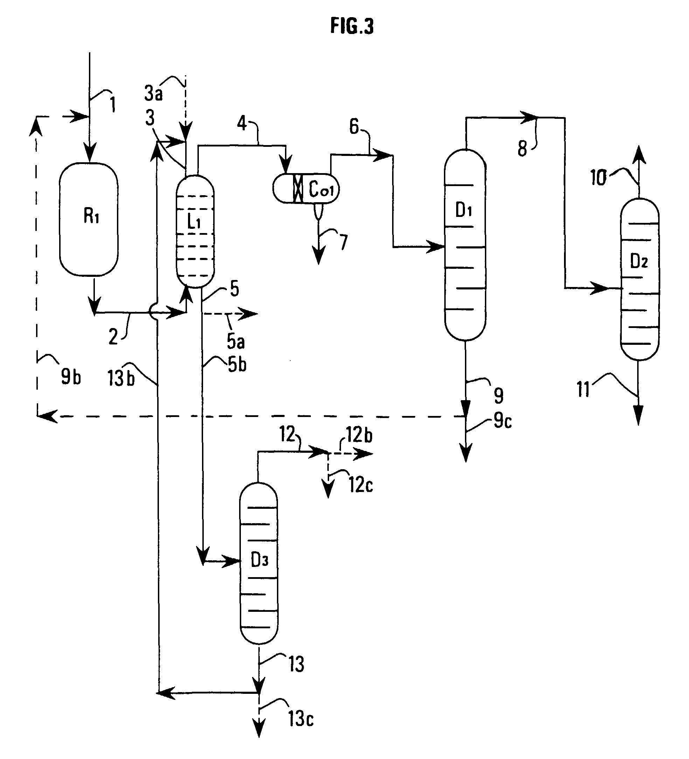

Selon une autre variante, le procédé de la présente invention comprend une étape e) (voir en particulier les figures 2 et 3) dans laquelle au moins une partie de la fraction (F1) issue de l'étape d) et de préférence la totalité de ladite fraction, est envoyée par la ligne 8 dans une zone de fractionnement (D2) à partir de laquelle on récupère par la ligne 10 une fraction (Lg1) contenant la majorité des composés légers initialement présents dans ladite partie et par la ligne 11 une fraction (Ohp3) contenant la majorité de l'oléfine tertiaire initialement contenue dans ladite partie. Ladite fraction (Ohp3) contient essentiellement de l'oléfine tertiaire purifiée.

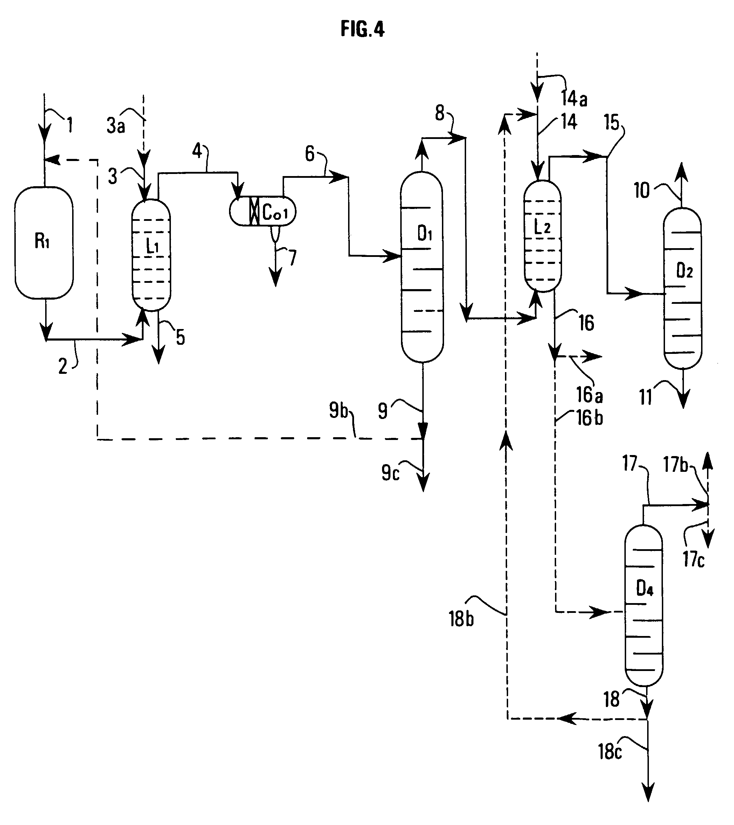

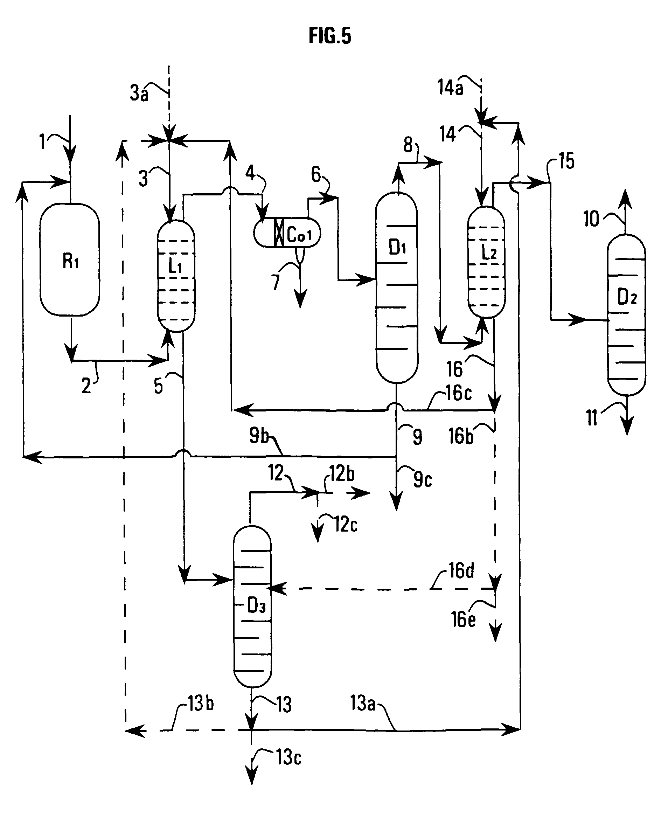

Selon une autre variante (voir en particulier les figures 4 et 5), le procédé de la présente invention comprend une étape g) dans laquelle au moins une partie de la fraction (F1) issue de l'étape d), et de préférence la totalité de ladite fraction, est envoyée par la ligne 8 dans une zone d'extraction (L2) par lavage à l'eau à partir de laquelle on obtient par la ligne 16 une fraction aqueuse (A2) contenant la majeure partie de l'alcool initialement présent dans ladite partie et par la ligne 15 une fraction (B2) contenant la majeure partie de l'oléfine tertiaire initialement présente dans ladite partie, ladite fraction (B2) contenant ladite oléfine tertiaire purifiée (Ohp4), de l'eau, et éventuellement des composés légers et étant sensiblement exempte d'alcool.

Selon une autre variante (voir en particulier les figures 4 et 5), le procédé de la présente invention comprend une étape h) dans laquelle au moins une partie de la fraction (B2) issue de l'étape g), et de préférence la totalité de ladite fraction, est envoyée par la ligne 15 dans une zone de fractionnement (D2) à partir de laquelle on récupère par la ligne 10 une fraction (Lg2) contenant la majorité des composés légers initialement présents dans ladite partie et par la ligne 11 une fraction (Ohp5) contenant la majorité de l'oléfine tertiaire initialement contenue dans ladite partie. Ladite fraction (Ohp5) contient essentiellement de l'oléfine tertiaire purifiée.

Selon une autre variante (voir en particulier figure 4), le procédé de la présente invention comprend une étape i) dans laquelle au moins une partie de la fraction aqueuse (A2) issue de l'étape g), et de préférence la totalité de ladite fraction, est envoyée par les lignes 16 et 16b dans une zone de fractionnement (D4) à partir de laquelle on récupère par la ligne 17 une fraction (G2) contenant la majeure partie de l'alcool initialement présent dans ladite partie et par la ligne 18 une fraction aqueuse (H2) débarrassée de la majeure partie de l'alcool initialement présent dans ladite partie. Selon ce mode de fonctionnement au moins une partie de la fraction (G2) obtenue à l'étape i) contenant de l'alcool peut être envoyée par la ligne 17c dans une zone de synthèse d'éther par réaction entre au moins une oléfine tertiaire et au moins un alcool. Il est également possible d'envoyer la totalité de cet alcool dans ladite zone de synthèse d'éther. On peut également récupérer par la ligne 17b en partie ou en totalité cet alcool pour d'autres usages. Il est également possible de recycler par les lignes 18b et 14 au moins une partie, voire la totalité, de la fraction aqueuse (H2) vers la zone (L2) d'extraction à l'eau de l'étape g) et/ou dans la zone (L1) d'extraction à l'eau de l'étape b) (non schématisé sur la figure 4). Ainsi au moins une partie de la fraction aqueuse (H2) est recyclé vers au moins une zone (L1) et/ou (L2) d'extraction à l'eau. Lorsque ce recyclage est partiel ou qu'il n'existe pas, cette partie de la fraction aqueuse (H2) peut être par exemple envoyée par la ligne 18c vers une zone de traitement des eaux usées.

Selon une autre variante (voir en particulier figure 5) du procédé de la présente invention, au moins une partie de la fraction (A2) issue de l'étape g) et de préférence la totalité de ladite fraction, est envoyée par les lignes 16, 16c et 3 dans la zone d'extraction (L1).

Selon une autre variante (voir en particulier figure 5) du procédé de la présente invention, l'étape f) est mise en oeuvre et au moins une partie de la fraction (A2) issue de l'étape g) est envoyée par les lignes 16, 16b et 16d vers la zone de fractionnement (D3) décrite ci-devant en liaison avec l'étape f).

Lorsque le recyclage de cette fraction (A2) est partiel ou qu'il n'existe pas, cette partie de la fraction aqueuse (A2) peut être par exemple envoyée, par les lignes 16, ou 16 et 16a (voir en particulier figure 4), ou 16, 16b et 16e (voir en particulier la figure 5), vers une zone de traitement des eaux usées.

Selon une autre variante (voir en particulier la figure 5) du procédé de la présente invention, l'étape f) est mise en oeuvre et au moins une partie de la fraction (H1) issue de l'étape f), et par exemple la totalité de ladite fraction, est recyclée vers au moins une zone d'extraction (L1) et/ou (L2), par exemple par les lignes 13, 13a et 14 dans la zone d'extraction (L2) par lavage à l'eau de l'étape g).

Selon une autre variante (voir en particulier la figure 5) du procédé de la présente invention, au moins une partie de la fraction (H1) issue de l'étape f) est recyclée par les lignes 13, 13b et 3 dans la zone d'extraction (L1), au moins une autre partie de la fraction (H1) issue de l'étape f) est recyclée par les lignes 13, 13a et 14 dans la zone d'extraction (L2) et éventuellement au moins encore une autre partie de la fraction (H1) issue de l'étape f) est purgée, par exemple envoyée par les lignes 13 et 13c vers une zone de traitement des eaux usées.

Dans le cas des variantes ci-devant où l'on recycle de l'eau dans la zone (L2), soit à partir de la fraction (H2) issue de l'étape i) soit à partir de la fraction aqueuse (H1) issue de l'étape f), soit encore à partir de ces deux fractions, la quantité d'eau employée dans la zone d'extraction (L2) est ajustée à l'aide d'au moins un moyen d'introduction d'eau d'appoint par la ligne 14a (voir en particulier figures 4 et 5) dans la zone (L2). Cet appoint d'eau permet en particulier de compenser les pertes d'eau dues à la saturation du flux hydrocarboné traité ainsi que le remplacement de l'eau éventuellement purgée.

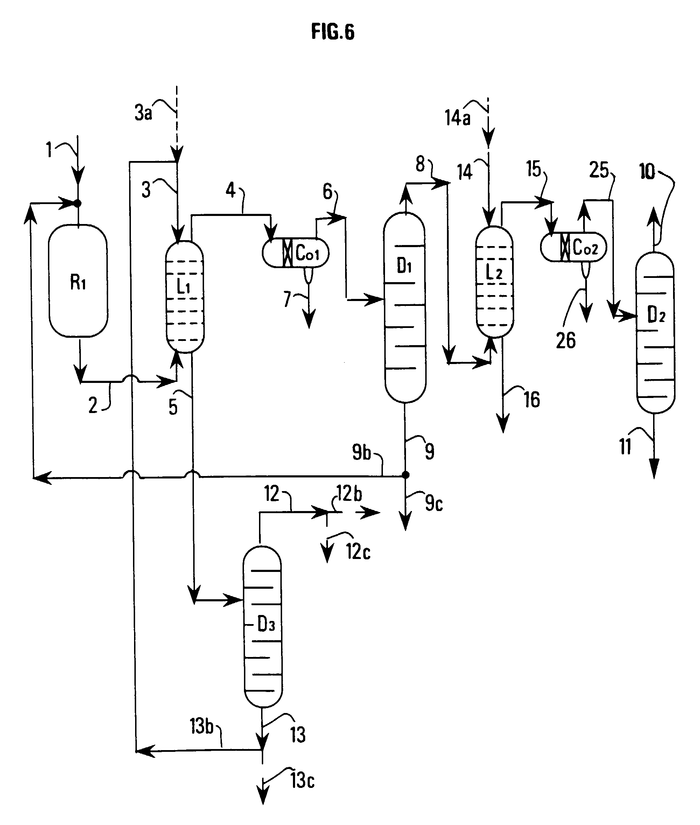

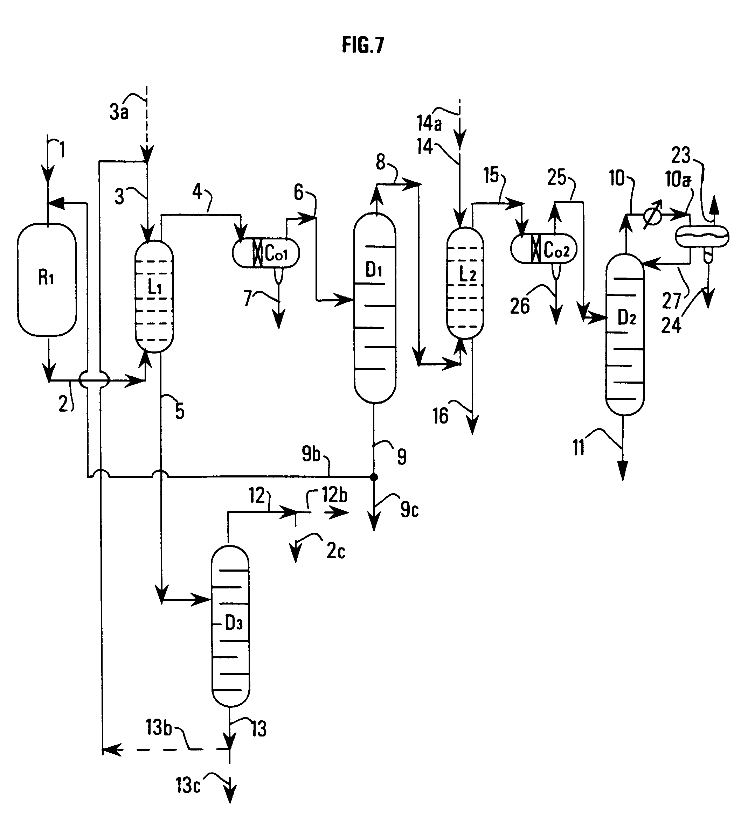

Selon une autre variante (voir en particulier les figures 6 et 7) le procédé de la présente invention comprendra une étape j) dans laquelle au moins une partie de la fraction (B2) issue de l'étape g) et de préférence la totalité de ladite fraction, est envoyée par la ligne 15 dans une zone de séparation (Co2) à partir de laquelle on récupère par la ligne 26 une fraction liquide aqueuse (La2) et par la ligne 25 une fraction liquide organique (Lb2) contenant la majeure partie de l'oléfine tertiaire initialement présente dans ladite partie, ladite fraction (Lb2) contenant ladite oléfine tertiaire purifiée (Ohp6) et des composés légers éventuels.

Selon une autre variante (voir en particulier les figures 6 et 7) le procédé de la présente invention comprendra une étape k) dans laquelle au moins une partie de la fraction (Lb2) issue de l'étape j), et de préférence la totalité de ladite fraction, est envoyée par la ligne 25 dans une zone de fractionnement (D2) à partir de laquelle on récupère par la ligne 10 une fraction (Lg3) contenant la majeure partie des composés légers initialement présents dans ladite partie et par la ligne 11 une fraction (Ohp7) contenant la majeure partie de l'oléfine tertiaire initialement contenue dans ladite partie. Ladite fraction (Ohp7) est essentiellement de l'oléfine tertiaire très pure.

Selon un mode particulier de mise en oeuvre (voir en particulier la figure 7) de la présente invention la colonne (D2) dans les diverses variantes décrites ci-devant (où au moins une étape e), h) ou k) est mise en oeuvre) comporte au moins un moyen permettant de récupérer à partir de la fraction [Lg1 - étape e) - Lg2 - étape h) - ou Lg3 - étape k)] une fraction légère (Lg4) sensiblement anhydre, ce moyen comportant le plus souvent un condenseur dans lequel ladite fraction gazeuse (Lg1, Lg2 ou Lg3) est introduite au moins en partie par la ligne 10 et ressort au moins en partie liquéfiée par la ligne 10a) permettra de scinder au moins une partie de la fraction (Lg1, Lg2 ou Lg3) en une fraction légère (Lg4) sensiblement anhydre et en une fraction aqueuse que l'on récupère par la ligne 24. Ce moyen sera par exemple un ballon séparateur munie d'au moins un moyen, par exemple une botte, permettant la décantation et le soutirage d'une fraction aqueuse. Dans ce cas la fraction légère sensiblement anhydre est habituellement scindée en une fraction gazeuse que l'on évacue par la ligne 23 par exemple à la torche et en une fraction liquide qui est au moins en partie renvoyée par la ligne 27 dans ladite zone de fractionnement.

Selon un autre mode de mise en oeuvre la fraction (Lg1, Lg2 ou Lg3) (ou la fraction légère (Lg4) sensiblement anhydre obtenue à partir de ladite fraction (Lg1, Lg2 ou Lg3) est au moins en partie envoyée dans une zone de craquage catalytique. Selon une autre variante, la fraction (Lg1 ou Lg2 ou Lg3 ou Lg4) est au moins en partie envoyée dans une zone de synthèse d'éther par réaction entre au moins un alcool et au moins une oléfine tertiaire.

Les diverses fractions aqueuses ((La1), (H1), (A2), (H2), (La2)) obtenues dans les diverses versions de mise en oeuvre de la présente invention peuvent être au moins en partie, voire en totalité, recyclées vers la zone de lavage (L1) lorsqu'il n'existe qu'une seule zone de lavage ou vers l'une ou l'autre des zones de lavage (L1) et (L2) lorsqu'il y a deux zones de lavage ou encore en partie vers l'une d'entre elles et en autre partie vers l'autre.

Les conditions de mise en oeuvre de l'étape a) de la présente invention sont des conditions classiques de décomposition d'éther alkylique tertiaire bien connues de l'homme du métier. Dans une forme préférée de réalisation cette étape a) sera mise en oeuvre sans addition d'eau supplémentaire au produit introduit dans la zone de décomposition. Il serait cependant possible d'ajouter une certaine quantité d'eau par exemple jusqu'à la limite de solubilité de l'eau dans l'éther que l'on souhaite décomposer. Habituellement les conditions de mise en oeuvre de cette étape a) sont choisies de manière à ce que la majeure partie de l'éther alkylique tertiaire se décompose pour donner un alcool et une oléfine tertiaire. Dans cette zone de décomposition la pression absolue est habituellement d'environ 1 environ 30 bar (1 bar est égal à 0,1 MPa), de préférence d'environ 1 à environ 12 bar, la température est habituellement comprise entre 50 °C et 300 °C et de préférence entre 100 °C et 250 °C, la VVH (vitesse spatiale horaire) est habituellement comprise entre 0,1 et 200 h⁻¹ et le plus souvent entre 0,5 et 100 h⁻¹. Dans cette zone on peut utiliser tous les catalyseurs acides bien connus de l'homme du métier. On préfère habituellement employer des catalyseurs acides solides. Ainsi le catalyseur peut être choisi dans le groupe formé par les résines acides organiques et les acides minéraux solides dans les conditions de la réaction de décomposition dudit éther. Parmi ces composés on emploie le plus souvent ceux choisis dans le groupe formé par les solides minéraux greffés comportant au moins un groupe organique de type alkyl-sulfonique, aryl-sulfonique ou alkylarylsulfonique. L'une des formes préférée de mise en oeuvre de cette étape a) utilise un catalyseur choisi dans le groupe formé par les polysiloxanes greffés par au moins un groupe alkyl-sulfonique.

Dans l'étape b), au moins une partie du produit (P) issu de l'étape a) est envoyée dans une zone d'extraction (L1) dans laquelle la quantité d'eau utilisée pour ce lavage est habituellement telle que le rapport volumique entre le volume de ladite quantité d'eau introduite dans ladite zone d'extraction et celui de laditie partie du produit (P) introduit dans ladite zone d'extraction (Vₑₐᵤ/V_{P}) soit d'environ 0,005 à environ 20. Le plus souvent cette quantité d'eau est telle que le rapport Vₑₐᵤ/V_{P} est d'environ 0,005 à environ 10, de préférence d'environ 0,01 à environ 5, et de manière encore plus préférée d'environ 0,02 à environ 1. En outre le débit d'eau dans cette zone de lavage (L1) peut être également régulé de manière plus fine en fonction du maintien d'un niveau de fond dans la zone de fractionnement D3 de l'eau et de l'alcool dans le cas de la présence d'une telle zone (D3). Ce niveau de fond peut être défini comme le niveau minimal nécessaire au bon fonctionnement de ladite zone (D3). Ce paramètre est un paramètre classique bien connu de l'homme du métier. Cette régulation est souvent faite en mode manuel par les opérateurs, mais il est possible que cette régulation soit effectuée par une boucle de régulation automatique dite LCR des initiales anglosaxonnes Level Control Regulation. Quel que soit le mode de régulation choisi, la quantité d'eau peut généralement être ajustée à l'aide d'au moins un moyen d'introduction d'eau d'appoint dans la zone (L1). Cet appoint d'eau permet en particulier de compenser les pertes d'eau dues à la saturation du flux hydrocarboné traité ainsi que le remplacement de l'eau éventuellement purgée. Cette zone d'extraction (L1) est habituellement une colonne à plateaux qui opère à une température d'environ 1 à environ 100 °C de préférence d'environ 10 à environ 60 °C. La pression absolue dans cette zone sera d'environ 1 à environ 20 bar, le plus souvent d'environ 1 à environ 15 bar, identique ou différente de celle régnant dans la zone de décomposition.

Dans l'étape c), une zone de séparation (Co1) d'une fraction liquide aqueuse (La1) et d'une fraction liquide organique (Lb1) est de préférence mise en oeuvre dans un appareil, dénommé coalesceur, dans lequel l'eau se rassemble à la partie inférieure de l'appareil par coalescence. Les conditions de température et de pression régnant dans cette zone sont dans les mêmes gammes que celles régnant dans l'étape b) d'extraction à l'eau. La pression (respectivement la température) peut être identique ou différente de celle régnant dans la zone (C1) de l'étape b). Dans cette zone on sépare ainsi l'eau libre contenue dans le produit issu de l'étape b). De plus cette zone a également le plus souvent une fonction de zone ou ballon de charge pour la zone de purification D1 de l'isobutène dans le cas de la présence d'une telle zone (D1). Tout autre moyen connu de l'homme du métier peut être employé dans le cadre de la présente invention. A titre d'exemple, on peut citer l'utilisation d'un absorbant ayant une sélectivité préférentielle pour l'une des fractions aqueuse ou organique.

Les conditions générales de mise en oeuvre de l'étape éventuelle d) de fractionnement dans une zone (D1), d'au moins une partie de la fraction (Lb1) issue de l'étape c), sont choisies en particulier en fonction des caractéristiques de l'éther résiduel et de l'oléfine tertiaire formée. L'homme du métier est à même de choisir ces conditions pour obtenir la séparation souhaitée entre une fraction contenant la majeure partie de l'éther résiduel et une fraction contenant la majeure partie de l'oléfine. Ainsi par exemple dans le cas de la décomposition du MTBE et de la formation d'isobutène et où la zone (D1) est une colonne de distillation, la pression absolue est d'environ 1 à environ 15 bar, le plus souvent d'environ 1 à environ 10 bar, identique ou différente de celle régnant dans la zone (Co1). La température de fond de la colonne dépend de la pression régnant dans ladite colonne et de la composition du produit de fond. Dans le cas d'une unité traitant 1 kg/h de MTBE, la zone de distillation (D1) comporte habituellement entre 3 et 80 plateaux théoriques et le plus souvent entre 5 et 50 plateaux théoriques.

L'étape e) éventuelle de fractionnement d'au moins une partie de la fraction (F1) contenant la majeure partie de l'oléfine tertiaire obtenue à l'étape d), dans une zone de fractionnement (D2) permet la récupération d'une fraction contenant la majeure partie de l'oléfine tertiaire initialement présente dans ladite partie de la fraction (F1) et une fraction (Lg1) contenant la majeure partie des composés légers initialement présent dans ladite partie. Les conditions générales de mise en oeuvre de cette étape e), dans le cas de la décomposition du MTBE et de formation d'isobutène sont les suivantes, dans le cas où la zone (D2) est une colonne de distillation, la pression absolue est d'environ 1 à environ 15 bar, le plus souvent d'environ 1 à environ 10 bar, identique ou différente de celle régnant dans la zone (D1). La température de fond de la colonne dépend à la fois de la pression régnant dans ladite colonne et de la pureté recherchée pour l'oléfine tertiaire récupérée en fond de colonne. Dans le cas d'une unité traitant 1 kg/h de MTBE la colonne de distillation (D2) comporte habituellement entre 2 et 80 plateaux théoriques et le plus souvent entre 3 et 60 plateaux théoriques.

L'étape f) de fractionnement de la fraction aqueuse (A1), contenant la majeure partie de l'alcool initialement présent dans le produit (P) dans une zone (D3), permet la séparation de ladite fraction (A1) en une fraction (G1) contenant la majeure partie de l'alcool initialement présent dans la fraction (A1) et en une fraction aqueuse (H1) débarrassée de la majeure partie de l'alcool initialement présent dans la fraction (A1). L'étape f) est habituellement réalisée dans une colonne à distiller (D3) sous une pression absolue d'environ 1 à environ 12 bar, de préférence d'environ 1 à environ 8 bar, identique ou différente de celle régnant dans la zone (L1). La température de fond de la colonne dépend en particulier de la pression régnant dans ladite colonne, elle est habituellement d'environ 50 à environ 300 °C et le plus souvent d'environ 65 à environ 200 °C. La colonne comporte habituellement environ 2 à environ 80 plateaux et le plus souvent environ 3 à environ 60 plateaux théoriques.

L'étape g) permet, dans une zone (L2), la purification d'au moins une partie de la fraction (F1) issue de l'étape d). La quantité d'eau utilisée pour le lavage à l'eau est habituellement telle que le rapport volumique entre le volume de ladite quantité d'eau introduite dans ladite zone d'extraction et celui de ladite partie de la fraction (F1) introduite dans ladite zone d'extraction (Vₑₐᵤ/V_{F1}) est d'environ 0,005 à environ 20. Le plus souvent cette quantité d'eau est telle que le rapport Vₑₐᵤ/V_{F1} est d'environ 0,005 à environ 10, de préférence d'environ 0,01 à environ 5, et de manière encore plus préférée d'environ 0,02 à environ 1. En outre le débit d'eau dans cette zone de lavage (L2) peut être également régulé de manière plus fine en fonction du maintien d'un niveau de fond, dans la zone de fractionnement D3 et/ou D4 dans le cas de la présence d'au moins une de ces zones, de l'eau et de l'alcool. Ce niveau de fond peut être défini comme le niveau minimal nécessaire au bon fonctionnement de la zone de fractionnement D3 et/ou D4. Ce paramètre est un paramètre classique bien connu de l'homme du métier. Cette régulation est souvent faite en mode manuel par les opérateurs, mais il est possible que cette régulation soit effectuée par une boucle de régulation automatique dite LCR des initiales anglosaxonnes Level Control Regulation. Quel que soit le mode de régulation choisi, la quantité d'eau est ajustée à l'aide d'au moins un moyen d'introduction d'eau d'appoint dans la zone (L2). Cet appoint d'eau permet en particulier de compenser les pertes d'eau dues à la saturation du flux hydrocarboné traité ainsi que le remplacement de l'eau éventuellement purgée. Cette zone d'extraction (L2) est habituellement une colonne à plateaux qui opère à une température d'environ 1 à environ 100 °C de préférence d'environ 10 à environ 60 °C. La pression absolue dans cette zone est d'environ 1 à environ 20 bar, le plus souvent d'environ 1 à environ 15 bar, identique ou différente de celle régnant dans la zone de fractionnement de l'étape d).

Les conditions générales de mise en oeuvre de l'étape h) de fractionnement d'au moins une partie de la fraction (B2) issue de l'étape g), dans une zone (D2), en une fraction liquide aqueuse (A2) et une fraction liquide organique (B2) sont dans les mêmes gammes que celles de l'étape g). Dans le cas de la décomposition du MTBE et de formation d'isobutène, la température de fond de la colonne utilisée généralement dans cette étape h) dépend à la fois de la pression régnant dans ladite colonne (D2) et de la pureté recherchée pour l'oléfine tertiaire récupérée en fond de colonne. Dans le cas d'une unité traitant 1 kg/h de MTBE, la colonne de distillation (D2) comporte habituellement entre 2 et 80 plateaux théoriques et le plus souvent entre 3 et 60 plateaux théoriques.

L'étape i) de fractionnement d'au moins une partie de la fraction aqueuse (A2), contenant la majeure partie de l'alcool initialement présent dans la fraction (F1), est opérée dans une zone (D4), dans laquelle on sépare ladite partie de fraction (A2) en une fraction (G2) contenant la majeure partie de l'alcool initialement présent dans la fraction (A2) et en une fraction aqueuse (H2) débarrassée de la majeure partie de l'alcool initialement présent dans la fraction (A2). L'étape i) est habituellement réalisée dans une colonne à distiller (D4) sous une pression absolue d'environ 1 à environ 12 bar, de préférence d'environ 1 à environ 8 bar, identique ou différente de celle régnant dans la zone d'extraction à l'eau de l'étape g). La température de fond de la colonne dépend en particulier de la pression régnant dans ladite colonne, elle est habituellement d'environ 50 à environ 300 °C et le plus souvent d'environ 65 à environ 200 °C. La colonne comporte habituellement environ 2 à environ 80 plateaux et le plus souvent environ 3 à environ 60 plateaux théoriques.

L'étape j) permet de séparer, dans une zone de séparation (Co2) une fraction liquide aqueuse (La2) et une fraction liquide organique (Lb2). L'étape j) est habituellement mise en oeuvre dans un appareil, dénommé coalesceur, dans lequel l'eau se rassemble à la partie inférieure de l'appareil par coalescence. Les conditions de température et de pression régnants dans cette zone sont dans les mêmes gammes que celles régnant dans la zone (L2). La pression et la température peuvent être identiques ou différentes de celles régnants dans la zone (D2). Dans cette zone, on sépare ainsi l'eau libre contenue dans le produit (B2) issu de l'étape g). De plus, cette zone a également le plus souvent une fonction de zone ou ballon de charge pour une zone de purification (D2) de l'isobutène dans le cas de la présence d'une telle zone (D2). Tout autre moyen connu de l'homme du métier peut être employé dans le cadre de la présente invention. A titre d'exemple d'un autre moyen utilisable, on peut citer l'utilisation d'un absorbant ayant une sélectivité préférentielle pour l'une des fractions aqueuse ou organique. Ladite fraction (Lb2) contient ladite oléfine tertiaire purifiée (Ohp6) et éventuellement des composés légers.

Les conditions générales de mise en oeuvre de l'étape k) de fractionnement, dans une zone (D2), d'au moins une partie de la fraction (Lb2) issue de l'étape j) en une fraction légère (Lg3) et une fraction liquide organique (Ohp7) sont choisies en particulier en fonction des caractéristiques de l'oléfine tertiaire formée. L'homme du métier est à même de choisir ces conditions pour obtenir la séparation souhaitée entre une fraction contenant la majeure partie des composés légers et une fraction contenant la majeure partie de l'oléfine. Ainsi par exemple dans le cas de la décomposition du MTBE et de la formation d'isobutène, la zone (D2) est une colonne de distillation et la pression absolue dans ladite colonne est d'environ 1 à environ 15 bar, le plus souvent d'environ 1 à environ 10 bar, identique ou différente de celle régnant dans la zone (Co2) de l'étape j). La température de fond de la colonne dépend de la pression régnant dans ladite colonne et de la composition du produit de fond. Dans le cas d'une unité traitant 1 kg/h de MTBE, la colonne de distillation (D2) comporte habituellement entre 3 et 80 plateaux théoriques et le plus souvent entre 5 et 50 plateaux théoriques.

L'exemple suivant illustre l'invention sans en limiter la portée.

### Exemple 1

L'exemple est mis en oeuvre à partir de la réalisation représentée sur la figure 2 (hors la ligne 9b qui n'est pas utilisée). On utilise un équipement de type pilote comprenant un réacteur tubulaire (R1), de 10 millilitres de volume, fonctionnant sous une pression relative de 7 bar, à une température moyenne de 160 °C contenant 3 grammes de catalyseur. On utilise un catalyseur commercial à base de polysiloxanes greffés par au moins un groupe alkyl sulfonique. On alimente le réacteur R1 par une charge contenant 100 % poids de MTBE, sous une VVH de 15 h⁻¹. Le tableau 1 présente la composition de la charge introduite dans le réacteur R1 de décomposition du MTBE et la composition du produit recueilli à la sortie du réacteur R1.

**Tableau 1**

| | Charge(% poids) | Effluent R1 (% poids) |
|---|---|---|
| MTBE | 100 | 10 |
| Isobutène | | 56,1 |
| Méthanol | | 32,1 |
| DME | | 0,5 |
| Dimères | | 1,1 |
| H2O | | 0,2 |

A l'aide du logiciel commercialisé par la société américaine SIMSCI (SIMulation SCIence INC.) sous la dénomination commerciale Pro II, on calcule les diverses sections de purification

Une colonne d'extraction par lavage à l'eau L1, qui est une colonne à plateaux et qui fonctionne à température 30 °C sous une pression relative de 12 bar utilisée dans l'étape b) du procédé de l'invention pour obtenir une fraction aqueuse (A1) et une fraction organique (B1),

Un système d'extraction de l'eau libre entraînée à l'étape b) avec la fraction organique (B1), du type coalesceur (Co1), qui fonctionne à 30 °C sous une pression relative de 12 bar, à partir duquel on obtient une fraction aqueuse (La1) et une fraction organique (Lb1).

Une colonne de distillation (D1), fonctionnant sous une pression relative de 7 bar, comportant 20 plateaux théoriques est utilisée à l'étape d) du procédé de l'invention pour obtenir un produit de fond (E1) et un produit de tête (F1),

Une colonne de distillation (D2), dernière étape de purification de l'isobutène, qui fonctionne sous une pression relative de 7 bar, comportant 20 plateaux théoriques utilisée à l'étape e) du procédé de l'invention pour obtenir un produit de fond (Ohp3) qui est de l'isobutène purifié et un produit de tête (Lg1) contenant des composés légers.

La colonne d'extraction par lavage à l'eau (L1) est alimentée par l'effluent (P) de (R1). À partir de cette colonne on récupère en fond une fraction aqueuse (A1) contenant la majorité du méthanol contenu dans le produit (P) issu du réacteur (R1) et en tête une fraction organique (B1) contenant la majorité de l'isobutène formé dans le réacteur (R1). Le produit (B1), contenant également une petite quantité d'eau entraînée à partir de la zone de lavage à l'eau (L1), est envoyé dans le système d'extraction de l'eau libre, du type coalesceur (Co1), à partir de laquelle on élimine l'eau par décantation. À partir de ce système, on récupère aussi une fraction essentiellement organique (Lb1). Cette fraction (Lb1) est envoyée dans une colonne à distiller (D1) à partir de laquelle on récupère en fond de colonne une fraction (E1) contenant principalement de l'éther non décomposé dans le réacteur (R1) et en tête de colonne une fraction (F1) contenant principalement de l'isobutène. La fraction (F1) est envoyée dans la colonne (D2) à partir de laquelle on obtient en tête une fraction légère (Lg1) contenant en particulier du DME (diméthyléther) et en fond de colonne une fraction (Ohp3) d'isobutène de haute pureté.

Les bilans matières obtenus sont donnés dans le tableau 2 et le tableau 3 ci-après.

**Tableau 2**

| | Effluent du réacteur R1 (% poids) | Eau de lavage colonne L1 | Fraction aqueuse A1 (g/h) | Fraction organique B1 (g/h) | fraction aqueuse La1 (g/h) | fraction organique Lb1 (g/h) |
|---|---|---|---|---|---|---|
| MTBE | 10 | | | 10 | | 10 |
| Isobutène | 56,1 | | | 56,1 | | 56,1 |
| Méthanol | 32,1 | | 32 | 0.1 | | 0,1 |
| DME | 0,5 | | | 0,5 | | 0,5 |
| Dimères | 1,1 | | | 1,1 | | 1,1 |
| H2O | 0,2 | 50 | 48 | 2,2 | 2,1 | 0,1 |
| Débit (g/h) | 100 | 50 | 80 | 70 | 2,1 | 67,9 |

**Tableau 3**

| | fraction F1 tête de D1 | fraction E1 fond de D1 | fraction Lg1 colonne D2 | fraction Ohp3 colonne D2 |
|---|---|---|---|---|
| MTBE | | 10 | | |
| Isobutène | 56,1 | | 1,6 | 54,5 |
| Méthanol | 0,1 | | 0,1 | |
| DME | 0,5 | | 0,45 | 0,05 |
| Dimères | | 1,1 | | |
| H2O | 0,1 | | 0,1 | |
| Débit (g/h) | 56,8 | 11,1 | 2,25 | 54,55 |
| Pureté de l'isobutène (%) | | | | >99,9 % |

Ainsi l'isobutène obtenu par le procédé selon l'invention est de très haute pureté.

### Exemple 2

Soit un équipement de type pilote comprenant un réacteur tubulaire R1, fonctionnant sous une pression relative de 7 bar, à une température moyenne de 140 °C. Le réacteur R1 contient du Deloxan ASP (catalyseur de type polysiloxane greffé par des groupements alkyl sulfonique). On alimente R1 par une charge contenant 100 % poids de TAME, sous une VVH de 6 h-1. Le produit recueilli à la sortie de R1 possède la composition donnée dans le tableau 4 :

**Tableau 4:**

| Section réactionnelle de décomposition du TAME | | |
|---|---|---|
| | Charge (% poids) | Effluent R1 (% poids) |
| TAME | 100 | 15 |
| Isoamylènes | | 58,2 |
| Méthanol | | 26,45 |
| DME | | 0,25 |
| Dimères | | 0,1 |

A l'aide du logiciel Pro II, les divers sections de purification peuvent être calculées comme dans l'exemple précédent, selon les spécifications requises pour chacun des flux sortants.

Nous nous contenterons ici de donner un bilan matière sommaire (tableau 5) pour illustrer une des mises en oeuvre de notre procédé (schéma analogue à celui de l'exemple 1, illustré par la figure 2, selon la deuxième colonne de purification D2).

**Tableau 5**

| | Effluent R1 | Eau de lavage | Fraction aqueuse A1 | Fraction organique B1 | Fraction organique Lb1 | Fraction F1 tête de D1 |
|---|---|---|---|---|---|---|
| TAME | 15 | | | 15 | 15 | |
| Isoamylènes | 58,2 | | | 58,2 | 58,2 | 58,2 |
| Méthanol | 26,45 | | 26,4 | 0,05 | 0,05 | 0,05 |
| DME | 0,25 | | | 0,25 | 0,25 | 0,25 |
| Dimères | 0,1 | | | 0,1 | 0,1 | |
| H2O | | 40 | 38 | 2 | 0,1 | 0,1 |
| Débit (g/h) | 100 | 40 | 64,4 | 75,6 | 73,7 | 58,6 |

On produit ainsi les isoamylènes avec un rendement minimum de 84 % (rendement pouvant être amélioré par le biais de recyclage de l'éther non converti, par exemple selon le mode illustré par la figure 2) et avec une pureté supérieure à 99 %.

## Revendications

1. Procédé de production d'oléfine tertiaire par décomposition d'éther alkylique tertiaire comprenant :
a) une étape de décomposition d'au moins un éther alkylique tertiaire dans une zone réactionnelle comprenant au moins un réacteur (R1) contenant un catalyseur de décomposition dudit éther, ladite étape étant effectuée dans des conditions permettant la décomposition au moins partielle dudit éther alkylique tertiaire en un produit (P) contenant au moins un alcool et au moins une oléfine tertiaire et éventuellement de l'éther non décomposé dans cette étape a) et des composés légers éventuels,
b) une étape b) de purification d'au moins une partie du produit (P), dans une zone d'extraction (L1) par lavage à l'eau, à partir de laquelle on obtient une fraction aqueuse (A1) contenant la majeure partie de l'alcool initialement présent dans ladite partie et une fraction (B1) contenant la majeure partie de l'oléfine tertiaire initialement présente dans ladite partie, ladite fraction (B1) contenant ladite oléfine tertiaire, de l'eau, éventuellement de l'éther et des composés légers éventuels et étant sensiblement exempte d'alcool ou en contenant une très faible proportion,
ledit procédé étant caractérisé en ce qu'il comporte une étape c) dans laquelle au moins une partie de la fraction (B1) issue de l'étape b) est envoyée dans une zone de séparation (Co1) à partir de laquelle on récupère une fraction liquide aqueuse (La1) et une fraction liquide organique (Lb1) contenant la majeure partie de l'oléfine tertiaire initialement présente dans la fraction (B1), ladite fraction (Lb1) contenant ladite oléfine tertiaire et éventuellement de l'éther et des composés légers éventuels.

2. Procédé selon la revendication 1 dans lequel au moins une partie de la fraction liquide aqueuse (La1) obtenue à l'étape c) est recyclée vers la zone (L1).

3. Procédé selon la revendication 1 ou 2 comprenant une étape f) dans laquelle au moins une partie de la fraction (A1) est envoyée dans une zone de fractionnement (D3) à partir de laquelle on récupère une fraction (G1) contenant la majeure partie de l'alcool initialement présent dans ladite partie et une fraction aqueuse (H1) débarrassée de la majeure partie de l'alcool initialement présent dans ladite partie.

4. Procédé selon la revendication 3 dans laquelle au moins une partie de la fraction (G1) est envoyie dans une zone de synthèse d'éther par réaction entre au moins un alcool et au moins une oléfine tertiaire.

5. Procédé selon la revendication 3 ou 4 dans laquelle au moins une partie de la fraction aqueuse (H1) est recyclée vers la zone (L1).

6. Procédé selon l'une des revendications 1 à 5 comprenant au moins un moyen d'introduction d'eau d'appoint dans la zone (L1).

7. Procédé selon l'une des revendications 1 à 6 comprenant une étape d) dans laquelle au moins une partie de la fraction organique (Lb1) est envoyée dans une zone de fractionnement (D1) à partir de laquelle on récupère une fraction (E1) contenant la majeure partie de l'éther initialement contenu dans ladite partie et une fraction (F1) contenant la majeure partie de l'oléfine tertiaire initialement contenue dans ladite partie.

8. Procédé selon la revendication 7 dans laquelle au moins une partie de la fraction (E1) est dans la zone (R1).

9. Procédé selon la revendication 7 ou 8 comprenant une étape e) dans laquelle au moins une partie de la fraction (F1) est envoyée dans une zone de fractionnement (D2) à partir de laquelle on récupère une fraction (Lg1) contenant la majorité des composés légers initialement présents dans ladite partie et une fraction (Ohp3) contenant la majeure partie de l'oléfine tertiaire initialement présente dans ladite partie.

10. Procédé selon la revendication 7 ou 8 comprenant une étape g) dans laquelle au moins une partie de la fraction (F1) est envoyée dans une zone d'extraction (L2) par lavage à l'eau à partir de laquelle on obtient une fraction aqueuse (A2) contenant la majeure partie de l'alcool initialement présent dans ladite partie et une fraction (B2) contenant la majeure partie de l'oléfine initialement présente dans ladite partie.

11. Procédé selon la revendication 10 comprenant une étape h) dans laquelle au moins une partie de la fraction organique (B2) est envoyée dans une zone de fractionnement (D2) à partir de laquelle on obtient une fraction (Ohp5) contenant la majeure partie de l'oléfine initialement présente dans ladite partie et une fraction (Lg2) contenant la majorité des composés légers initialement présents dans ladite partie.

12. Procédé selon la revendication 10 ou 11 comprenant une étape i) dans laquelle au moins une partie de la fraction aqueuse (A2) issue de l'étape g) est envoyée dans une zone de fractionnement (D4) à partir de laquelle on récupère une fraction (G2) contenant la majeure partie de l'alcool initialement présent dans ladite partie et une fraction aqueuse (H2) débarrassée de la majeure partie de l'alcool initialement présent dans ladite partie.

13. Procédé selon la revendication 12 dans laquelle au moins une partie de la fraction (G2) est envoyée dans une zone de synthèse d'éther par réaction entre au moins un alcool et au moins une oléfine tertiaire.

14. Procédé selon la revendication 12 ou 13 dans laquelle au moins une partie de la fraction aqueuse (H2) est recyclée vers au moins une zone, (L1) et/ou (L2) d'extraction à l'eau.

15. Procédé selon l'une des revendications 10 à 14 dans lequel au moins une partie de la fraction aqueuse (A2) est recyclée vers la zone (L1).

16. Procédé selon l'une des revendications 10 à 15 dans lequel l'étape f) est mise en oeuvre et au moins une partie de la fraction aqueuse (A2) est envoyée dans la zone de fractionnement (D3) de l'étape f).

17. Procédé selon l'une des revendications 10 à 16 dans lequel l'étape f) est mise en oeuvre et au moins une partie de la fraction aqueuse (H1) issue de l'étape f) est recyclée vers au moins une zone d'extraction (L1) et/ou (L2).

18. Procédé selon l'une des revendications 10 à 17 comprenant au moins un moyen d'introduction d'eau d'appoint dans la zone (L2).

19. Procédé selon les revendications 10, et 12 à 18 comprenant une étape j) dans laquelle au moins une partie de la fraction (B2) issue de l'étape g) est envoyée dans une zone de séparation (Co2) à partir de laquelle on récupère une fraction liquide aqueuse (La2) et une fraction liquide organique (Lb2) contenant la majeure partie de l'oléfine tertiaire initialement présente dans ladite partie, ladite fraction (Lb2) contenant ladite oléfine tertiaire purifiée et des composés légers éventuels.

20. Procédé selon la revendication 19 comprenant une étape k) dans laquelle au moins une partie de la fraction (Lb2) issue de l'étape j) est envoyée dans une zone de fractionnement (D2) à partir de laquelle on récupère une fraction (Ohp7) contenant la majeure partie de l'oléfine initialement présente dans ladite partie et une fraction (Lg3) contenant la majorité des composés légers initialement présents dans ladite fraction (Lb2).

21. Procédé selon l'une des revendications 9 à 20 dans lequel une étape e), h) ou k) est mise en oeuvre et la zone de fractionnement (D2) à partir de laquelle on récupère une fraction (Lg1 ou Lg2 ou Lg3) comporte au moins un moyen permettant d'obtenir une fraction légère (Lg4) sensiblement anhydre.

22. Procédé selon la revendication 21 dans lequel ledit moyen permettant d'obtenir ladite fraction (Lg4) comporte au moins un condenseur permettant de condenser au moins en partie la fraction (Lg1 ou Lg2 ou Lg3).

23. Procédé selon l'une des revendications 21 ou 22 dans lequel ledit moyen permet de récupérer une fraction aqueuse et de recycler une fraction liquide organique dans ladite zone de fractionnement (D2).

24. Procédé selon l'une des revendications 21 à 23 dans lequel ladite fraction (Lg4) est au moins en partie envoyée dans une zone de synthèse d'éther par réaction entre au moins un alcool et au moins une oléfine tertiaire.

25. Procédé selon l'une des revendications 9 à 20 dans lequel une étape e), h) ou k) est mise en oeuvre et la fraction (Lg1 ou Lg2 ou Lg3) issue de la zone de fractionnement (D2) est au moins en partie envoyée dans une zone de synthèse d'éther par réaction entre au moins un alcool et au moins une oléfine tertiaire.

## Patentansprüche

1. Verfahren zur Herstellung von tertiärem Olefin durch Zersetzung von tertiärem Alkylether umfassend:
a) eine Stufe der Zersetzung wenigstens eines tertiären Alkylethers in einer Reaktionszone, die wenigstens einen Reaktor (R1) umfasst, der über einen Katalysator zur Zersetzung dieses Ethers verfügt, wobei diese Stufe unter Bedingungen durchgeführt wird, welche die wenigstens teilweise Zersetzung dieses tertiären Alkylethers in ein Produkt (P) ermöglichen, das wenigstens einen Alkohol und wenigstens ein tertiäres Olefin und gegebenenfalls in dieser Stufe a) nicht zersetzten Ether und eventuelle leichte Verbindungen enthält,
b) eine Reinigungsstufe b) wenigstens eines Teils des Produktes (P) in einer Zone (L1) der Extraktion durch Waschen mit Wasser, von der man eine wässrige Fraktion (A1) erhält, welche den größeren Teil des anfänglich in diesem Teil vorliegenden Alkohols enthält, und eine Fraktion (B1), die den größeren Teil des anfänglich in diesem Teil vorliegenden tertiären Olefins enthält, wobei diese Fraktion (B1) dieses tertiäre Olefin, Wasser, ggf. Ether und eventuelle leichte Verbindungen enthält und im wesentlichen frei von Alkohol ist oder ihn in einem sehr geringen Verhältnis enthält,
wobei sich dieses Verfahren dadurch auszeichnet, dass es eine Stufe c) umfasst, in der wenigstens ein Teil der aus der Stufe b) stammenden Fraktion (B1) in eine Trennzone (Co1) geleitet wird, aus der man eine flüssige, wässrige Fraktion (La1) und eine flüssige organische Fraktion (Lb1) gewinnt, welche den größeren Teil des anfänglich in der Fraktion (B1) vorliegenden tertiären 0lefins enthält, wobei diese Fraktion (Lb1) dieses tertiäre Olefin und ggf. Ether und eventuelle leichte Verbindungen enthält.

2. Verfahren nach Anspruch 1, bei dem die wässrige, flüssige, in Stufe c) erhaltene Fraktion (La1) zur Zone (L1) rezykliert wird.

3. Verfahren nach Anspruch 1 oder 2, welches eine Stufe f) umfasst, in der wenigstens ein Teil der Fraktion (A1) in eine Fraktionierungszone (D3) geleitet wird, von der man eine Fraktion (G1) gewinnt, welche den größeren Teil des anfänglich in diesem Teil vorliegenden Alkohols enthält, und eine wässrige Fraktion (H1), die großteils vom anfänglich vorliegenden Alkohol befreit ist.

4. Verfahren nach Anspruch 3, bei dem wenigstens ein Teil der Fraktion (G1) in eine Zone der Ethersynthese durch Reaktion zwischen wenigstens einem Alkohol und wenigstens einem tertiären Olefin geleitet wird.

5. Verfahren nach Anspruch 3 oder 4, bei dem wenigstens ein Teil der wässrigen Fraktion (H1) zur Zone (L1) rezykliert wird.

6. Verfahren nach einem der Ansprüche 1 bis 5, das wenigstens ein Mittel zur Einfuhr zusätzlichen Wassers in die Zone (L1) umfasst.

7. Verfahren, nach einem der Ansprüche 1 bis 6, welches eine Stufe d) umfasst, in der wenigstens ein Teil der organischen Fraktion (Lb1) in eine Fraktionierungszone (D1) geleitet wird, von der man eine Fraktion (E1) gewinnt, welche den größeren Teil des anfänglich in diesem Teil vorliegenden Ethers enthält, und eine Fraktion (F1), welche den größeren Teil des anfänglich in diesem Teil vorliegenden tertiären Olefins enthält.

8. Verfahren nach Anspruch 7, bei dem wenigstens ein Teil der Fraktion (E1) in die Zone (R1) rezykliert wird.

9. Verfahren nach Anspruch 7 oder 8, welches eine Stufe e) umfasst, in der zumindest ein Teil der Fraktion (F1) in eine Fraktionierungszone (D2) geleitet wird, von der man eine Fraktion (Lg1) gewinnt, welche den Großteil der anfänglich in diesem Teil vorliegenden leichten Verbindungen enthält, und eine Fraktion (Ohp3), welche den größeren Teil des anfänglich in diesem Teil vorliegenden tertiären Olefins enthält.

10. Verfahren nach Anspruch 7 oder 8, welches eine Stufe g) umfasst, in der zumindest ein Teil der Fraktion (F1) in eine Zone (L2) der Extraktion durch Waschen mit Wasser geleitet wird, von der man eine wässrige Fraktion (A2) gewinnt, welche den größeren Teil des anfänglich in diesem Teil vorliegenden Alkohols enthält, und eine Fraktion (B2), welche den größeren Teil des anfänglich in diesem Teil vorliegenden Olefins enthält.

11. Verfahren nach Anspruch 10, welches eine Stufe h) umfasst, in der zumindest ein Teil der organischen Fraktion (B2) in eine Fraktionierungszone (D2) geleitet wird, von der man eine Fraktion (Ohp5) gewinnt, welche Großteil des anfänglich in diesem Teil vorliegenden Olefins enthalt, und eine Fraktion (Lg2), welche den größeren Teil der anfänglich in diesem Teil vorliegenden leichten Verbindungen enthält.

12. Verfahren nach Anspruch 10 oder 11, welches eine Stufe i) umfasst, in der zumindest ein Teil der aus Stufe g) stammenden wässrigen Fraktion (A2) in eine Fraktionierungszone (D4) geleitet wird, von der man eine Fraktion (G2) gewinnt, welche den größeren Teil des anfänglich in diesem Teil vorliegenden Alkohols enthält, und eine wässrige Fraktion (H2), welche großteils vom anfänglich in diesem Teil vorliegenden Alkohol befreit ist.

13. Verfahren nach Anspruch 12, bei dem wenigstens ein Teil der Fraktion (G2) in eine Zone der Synthese von Ether durch Reaktion zwischen wenigstens einem Alkohol und wenigstens einem tertiären Olefin geleitet wird.

14. Verfahren nach Anspruch 12 oder 13, bei dem wenigstens ein Teil der wässrigen Fraktion (H2) zu wenigstens einer Zone (L1) und /oder (L2) der Extraktion mit Wasser rezykliert wird.

15. Verfahren nach einem der Ansprüche 10 bis 14, bei dem wenigstens ein Teil der wässrigen Fraktion (A2) zur Zone (L1) rezykliert wird.

16. Verfahren nach einem der Ansprüche 10 bis 15, bei dem die Stufe f) durchgeführt wird und wenigstens ein Teil der wässrigen Fraktion (A2) in die Fraktionierungszone (D3) der Stufe f) geleitet wird.

17. Verfahren nach einem der Ansprüche 10 bis 16, bei dem die Stufe f) durchgeführt wird und wenigstens ein Teil der wässrigen Fraktion (H1) aus Stufe f) zu wenigstens einer Extraktionszone (L1) und /oder (L2) rezykliert wird.

18. Verfahren nach einem der Ansprüche 10 bis 17, das wenigstens ein Mittel zur Einfuhr zusätzlichen Wassers in die Zone (L2) umfasst.

19. Verfahren nach einem der Ansprüche 10 und 12 bis 18, das eine Stufe j) umfasst, in der wenigstens ein Teil der Fraktion (B2) aus Stufe g) in eine Trennzone (Co2) geleitet wird, von der man eine wässrige flüssige Fraktion (La2) und eine organische flüssige Fraktion (Lb2) gewinnt, welche den Großteil des anfänglich in diesem Teil vorliegenden tertiären Olefins enthält, wobei diese Fraktion (Lb2) dieses gereinigte tertiäre Olefin und eventuelle leichte Verbindungen enthält.

20. Verfahren nach Anspruch 19, welches eine Stufe k) umfasst, in der zumindest ein Teil der aus Stufe j) stammenden wässrigen Fraktion (Lb2) in eine Fraktionierungszone (D2) geleitet wird, von der man eine Fraktion (Ohp7), welche den größeren Teil des anfänglich in diesem Teil vorliegenden Olefins enthält, und eine Fraktion (Lg3) gewinnt, welche den Großteil der anfänglich in dieser Fraktion (Lb2) vorliegenden leichten Verbindungen enthält.

21. Verfahren nach einem der Ansprüche 9 bis 20, bei dem eine Stufe e), h) oder k) und eine Fraktionierungszone (D2) verwirklicht wird, von der man eine Fraktion (Lg1 oder Lg2 oder Lg3) gewinnt, welche zumindest ein Mittel umfasst, um eine leichte, im wesentlichen wasserfreie Fraktion (Lg4) zu erhalten.

22. Verfahren nach Anspruch 21, bei dem dieses Mittel, welches es erlaubt, diese Fraktion (Lg4) zu erhalten, wenigstens einen Kondensator umfasst, der es erlaubt, wenigstens teilweise die Fraktion (Lg1 oder Lg2 oder Lg3) zu kondensieren.

23. Verfahren nach einem der Ansprüche 21 oder 22, bei dem dieses Mittel es erlaubt, eine wässrige Fraktion zu gewinnen und eine organische, flüssige Fraktion in diese Fraktionierungszone (D2) zu rezyklieren.

24. Verfahren nach einem der Ansprüche 21 bis 23, bei dem diese Fraktion (Lg4) wenigstens zum Teil in eine Zone der Ethersynthese durch Reaktion zwischen wenigstens einem Alkohol und wenigstens einem tertiären Olefin geleitet wird.

25. Verfahren nach einem der Ansprüche 9 bis 20, bei dem eine Stufe e), h) oder k) ausgeführt wird und die aus der Fraktionierungszone (D2) stammende Fraktion (Lg1 oder Lg2 oder Lg3) wenigstens zum Teil in eine Zone der Ethersynthese durch Reaktion zwischen wenigstens einem Alkohol und wenigstens einem tertiären Olefin geleitet wird.

## Claims

1. A process for producing a tertiary olefin by decomposing a tertiary alkyl ether, comprising:
a) a step for decomposing at least one tertiary alkyl ether in a reaction zone comprising at least one reactor (R1) containing a catalyst for decomposing said ether, said step being carried out under conditions which can at least partially decompose said tertiary alkyl ether to a product (P) containing at least one alcohol and at least one tertiary olefin and possibly ether which has not been decomposed in step a) and possibly light compounds;
b) a step b) for purifying at least a portion of product (P) in a water washing extraction zone (L1) from which an aqueous fraction (A1) containing the major portion of the alcohol initially present in said portion and a fraction (B1) containing the major portion of the tertiary olefin initially present in said portion are obtained, said fraction (B1) containing said tertiary olefin, water, possibly ether and possibly light compounds and being substantially free of alcohol or containing a very small proportion thereof;
said process being characterized in that it comprises a step c) in which at least a portion of fraction (B1) from step b) is sent to a separation zone (Co1), from which a liquid aqueous fraction (La1) and a liquid organic fraction (Lb1) containing the major portion of the tertiary olefin initially present in said portion of the fraction (B1) are recovered, said fraction (Lb1) containing said tertiary olefin and possibly ether and possibly light compounds.

2. A process according to claim 1, in which at least a portion of the liquid aqueous fraction (La1) obtained from step c) is recycled to zone (L1).

3. A process according to claim 1 or claim 2, comprising a step f) in which at least a portion of fraction (A1) is sent to a fractionation zone (D3) from which a fraction (G1) containing the major portion of the alcohol initially present in said portion and an aqueous fraction (H1) which is free of the major portion of the alcohol initially present in said portion are recovered.

4. A process according to claim 3, in which at least a portion of fraction (G1) is sent to a zone for synthesising ether by reaction between at least one alcohol and at least one tertiary olefin.

5. A process according to claim 3 or claim 4, in which at least a portion of the aqueous fraction (H1) is recycled to zone (L1).

6. A process according to any one of claims 1 to 5, comprising at least one means for introducing makeup water into zone (L1).

7. A process according to any one of claims 1 to 6, comprising a step d) in which at least a portion of the organic fraction (Lb1) is sent to a fractionation zone (D1) from which a fraction (E1) containing the major portion of the ether initially contained in said portion and a fraction (F1) containing the major portion of the tertiary olefin initially contained in said portion are recovered.

8. A process according to claim 7, in which at least a portion of the fraction (E1) is in zone (R1).

9. A process according to claim 7 or claim 8, comprising a step e) in which at least a portion of fraction (F1) is sent to a fractionation zone (D2) from which a fraction (Lg1) containing the majority of the light compounds initially present in said portion and a fraction (Ohp3) containing the majority of the tertiary olefin initially contained in said portion are recovered.

10. A process according to claim 7 or claim 8, comprising a step g) in which at least a portion of fraction (F1) is sent to a water washing extraction (L2) from which an aqueous fraction (A2) containing the major portion of the alcohol initially present in said portion and a fraction (B2) containing the major portion of the tertiary olefin initially present in that portion are obtained.

11. A process according to claim 10, comprising a step h) in which at least a portion of organic fraction (B2) is sent to a fractionation zone (D2) from which a fraction (Ohp5) containing the major portion of the tertiary olefin initially present in said portion and a fraction (Lg2) containing the majority of the light compounds initially present in said portion are recovered.

12. A process according to claim 10 or claim 11, comprising a step i) in which at least a portion of the aqueous fraction (A2) from step g) is sent to a fractionation zone (D4) from which a fraction (G2) containing the major portion of the alcohol initially present in said portion and an aqueous fraction (H2) which is free of the major portion of the alcohol initially present in that portion are recovered.

13. A process according to claim 12, in which at least a portion of the fraction (G2) is sent to a zone for synthesising ether by reaction between at least one alcohol and at least one tertiary olefin.

14. A process according to claim 12 or claim 13, in which at least a portion of aqueous fraction (H2) is recycled to at least one water extraction zone, (L1) and/or (L2).

15. A process according to any one of claims 10 to 14, in which at least a portion of the aqueous fraction (A2) is recycled to zone (L1).

16. A process according to any one of claims 10 to 15, in which step f) is carried out and at least a portion of the aqueous fraction (A2) is sent to the fractionation zone (D3) of step f).

17. A process according to any one of claims 10 to 16, in which step f) is carried out and at least a portion of the aqueous fraction (H1) from step f) is recycled to at least one extraction zone (L1) and/or (L2).

18. A process according to any one of claims 10 to 17, comprising at least one means for introducing makeup water into zone (L2).

19. A process according to claims 10 and 12 to 18, comprising a step j) in which at least a portion of fraction (B2) from step g) is sent to a separation zone (Co2) from which a liquid aqueous fraction (La2) and a liquid organic fraction (Lb2) containing the major portion of the tertiary olefin initially present in said portion are recovered, said fraction (Lb2) containing said purified tertiary olefin and any light compounds.

20. A process according to claim 19, comprising a step k) in which at least a portion of fraction (Lb2) from step j) is sent to a fractionation zone (D2) from which a fraction (Ohp7) containing the major portion of the olefin initially present in said portion and a fraction (Lg3) containing the majority of the light compounds initially present in said fraction (Lb2) are recovered.

21. A process according to any one of claims 9 to 20, in which one of steps e), h) or k) is carried out and the fractionation zone (D2) from which a fraction (Lg1 or Lg2 or Lg3) is recovered comprises at least one means for obtaining a substantially anhydrous light fraction (Lg4).

22. A process according to claim 21, in which said means for obtaining said fraction (Lg4) comprises at least one condenser for condensing at least a portion of the fraction (Lg1 or Lg2 or Lg3).

23. A process according to claim 21 or claim 22, in which said means can recover an aqueous fraction and recycle a liquid organic fraction to said fractionation zone (D2).

24. A process according to any one of claims 21 to 23, in which at least part of said fraction (Lg4) is sent to a zone for synthesising ether by reaction between at least one alcohol and at least one tertiary olefin.

25. A process according to any one of claims 9 to 20, in which one of steps e), h) or k) is carried out and at least part of the fraction (Lg1 or Lg2 or Lg3) from the fractionation zone (D2) is sent to a zone for synthesising ether by reaction between at least one alcohol and at least one tertiary olefin.
